(19) Europäisches Patentamt
European Patent Office
Office européen des brevets

(11) Publication number: **0 435 367 A1**

# (12) EUROPEAN PATENT APPLICATION

(21) Application number: **90203286.1**

(51) Int. Cl.[5]: **C07C 2/30**, B01J 31/28

(22) Date of filing: **12.12.90**

(30) Priority: **28.12.89 GB 8929269**

(43) Date of publication of application: **03.07.91 Bulletin 91/27**

(84) Designated Contracting States: **DE ES FR GB IT**

(71) Applicant: **SHELL INTERNATIONALE RESEARCH MAATSCHAPPIJ B.V.**
**Carel van Bylandtlaan 30**
**NL-2596 HR Den Haag(NL)**

(72) Inventor: **Doyle, Michael John**
**Badhuisweg 3**
**NL-1031 CM Amsterdam(NL)**
Inventor: **Clark, Jonathan Malcolm Thonger**
**Badhuisweg 3**
**NL-1031 CM Amsterdam(NL)**

(74) Representative: **Aalbers, Onno et al**
**P.O. Box 302**
**NL-2501 CH The Hague(NL)**

## (54) Process and catalyst composition for the preparation of linear alpha olefins.

(57) A catalyst composition, suitable for use in the oligomerization of ethene to substantially linear alpha olefins, comprises a zirconium or hafnium compound, an aluminium compound and a compound of the general formula (I)

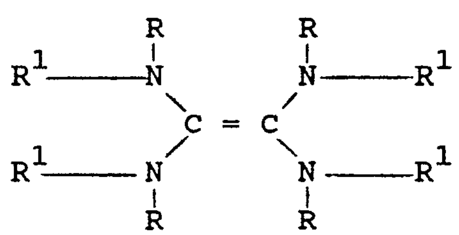

(I)

in which each R, which may be the same or different, represents an optionally substituted alkyl, cycloalkyl, aralkyl or aryl group, or groups R on cis nitrogen atoms may together form an aliphatic, cycloaliphatic or aromatic bridging group which may contain heteroatoms; and each $R^1$, which may be the same or different, represents an optionally substituted alkyl, cycloalkyl, aralkyl or aryl group, or groups $R^1$ on adjacent nitrogen atoms may together form an aliphatic, cycloaliphatic or aromatic bridging group which may contain hetero atoms; or adjacent groups R and $R^1$ together with the nitrogen to which they are attached may form an optionally substituted heterocyclic ring.

EP 0 435 367 A1

# PROCESS AND CATALYST COMPOSITION FOR THE PREPARATION OF LINEAR ALPHA OLEFINS

The present invention relates to a process for the preparation of linear alpha olefins and to catalyst compositions suitable for use in the process.

Linear alpha olefins are known to be valuable intermediates, for example in the preparation of polyolefins, detergents and lubricant additives. Processes for preparing for example $C_6$-$C_{20}$ olefins from lower olefins such as ethene by oligomerization in the presence of a Ziegler catalyst system are already known. Examples of such processes are to be found in EP-A-295960, US 4486615 and EP-A-241596. EP-A-295960 describes an oligomerization process in which a two component catalyst system is used which comprises an alkyl metal compound and a soluble adduct of a zirconium tetrahalide with an organic compound such as an acetate. US 4 486 615 describes the use in olefin oligomerization of the reaction product of a zirconium compound with an organo aluminium compound and describes the possibility of including a Lewis base in the catalyst system, so as to increase catalyst activity and molecular weight of the product olefins, the preferred Lewis base being triethylamine. EP-A-241596 purports to provide an improvement on the catalyst system described in US 4 486 615 in terms of product purity, the improved catalyst composition comprising a zirconium halide, an organoaluminium compound and a Lewis base, which Lewis base is specifically selected from thioethers, alkyl disulphides, thiophenes, thiourea, sulphides, phosphines and primary amines (eg. aniline).

Surprisingly, it has now been found that certain N-substituted olefins which contain an electron rich double bond can be successfully employed as an activating component in a Ziegler catalyst system for olefin oligomerization in place of the previously proposed Lewis bases. Such N-substituted olefins are known per se and have previously been employed in reactions involving nucleophilic attack at the carbons of the electron rich double bond, see, for example, the use of such electron rich olefins in the preparation of cyclic aminals described in US 4000152, the preparation of certain amino substituted ethylenes as described by Schönberg et al, Chem. Ber., 120, (1987), 1589-1591 and the participation of such electron rich olefins in a variety of reactions which in all cases involve the reactive olefinic carbon atoms (rather than the heterocyclic nitrogen atoms) as described by Hoffman, Angew. Chem. Internat. Edit., 7, (1968) No 10, 754-765 and Wiberg, Angew. Chem. Internat, Edit., 7, (1968) No 10, 766 to 779. Furthermore, it has been found that the inclusion of such N-substituted olefins as a catalyst component in olefin oligomerization may result in a shift in the product slate obtained towards linear alpha olefins of somewhat lower molecular weight. This ability to tailor the product slate to somewhat lower molecular weight oligomers is desirable in that such oligomers are valuable intermediates in the preparation of linear low density polyethylenes and certain lubricants.

According to the present invention, there is provided a catalyst composition comprising a zirconium or hafnium compound, an aluminium compound and a compound of the general formula (I)

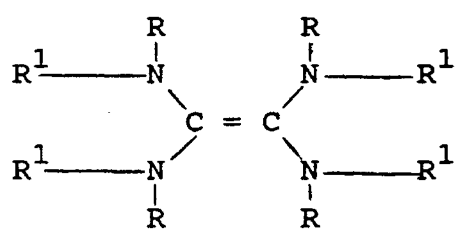

(I)

in which each R, which may be the same or different, represents an optionally substituted alkyl, cycloalkyl, aralkyl or aryl group, or groups R on cis nitrogen atoms may together form an aliphatic, cycloaliphatic or aromatic bridging group which may contain heteroatoms; and each $R^1$, which may be the same or different, represents an optionally substituted alkyl, cycloalkyl, aralkyl or aryl group, or groups $R^1$ on adjacent nitrogen atoms may together form an aliphatic, cycloaliphatic or aromatic bridging group which may contain hetero atoms; or adjacent groups R and $R^1$ together with the nitrogen to which they are attached may form an optionally substituted heterocyclic ring. Optional substituents include halogen and nitro.

Examples of such compounds are to be found in the papers by Schönberg et. al., Hoffman and Wiberg as mentioned above. However a preferred group of compounds of formula I are those of general formula (II):

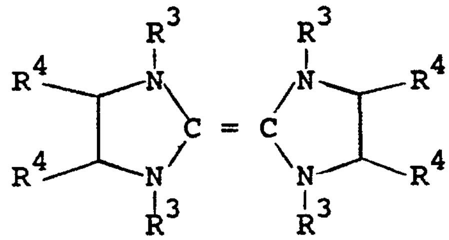

(II)

in which each $R^3$, which may be the same or different, represents an optionally substituted alkyl, cycloalkyl, or aryl group, or groups $R^3$ on different rings may be linked to form an optionally substituted alkylene linkage and each $R^4$, which may be the same or different, represents a hydrogen atom or an optionally substituted alkyl group.

Preferred compounds of general formula (II) are those in which each $R^3$ is an alkyl group or an optionally substituted phenyl group. Preferably each $R^4$ represents a hydrogen atom. Preferred compounds are 1,1',3,3'-tetraphenyl-2,2'-biimidazolidinylidene and 1,1'3,3'-tetra-p-tolyl-2,2'-biimidazolidinylidene.

Compounds of formulae (I) and (II) are known, as described in the references cited above and may be prepared, for example, by the methods described by Wanzlick et al, Chem. Ber., 1963, 96, 1208-1212.

The zirconium or hafnium compound may be an inorganic metal compound such as a halide, preferably a chloride, bromide, chlorobromide or iodide. Alternatively, an organozirconium or hafnium compound may be employed such as an alkoxide, alkoxyhalide, cycloalkylhalide, aryl halide, aralkylhalide, carboxylate or a benzyl, phenoxy, cycloalkyl, aryl or aralkyl derivative. A preferred compound is zirconium tetrachloride.

The aluminium compound may suitably be an inorganic compound such as aluminium trichloride or an organo aluminium compound, especially an organoaluminium halide such as a dialkyl aluminium chloride or an alkyl aluminium sesquichloride. Alternative aluminium compounds are trialkyl aluminium, tricycloalkyl aluminium, tribenzyl aluminium, cycloalkyl aluminium sesquichloride, alkyl-, cycloalkyl- and benzyl aluminium dichlorides and dicycloalkyl- and dibenzylalkyl aluminium chloride. A preferred compound is diethylaluminium chloride.

Mixtures of compounds of formula (I), mixtures of aluminium compounds and mixtures of zirconium and/or hafnium compounds may be used.

The relative proportion of the catalyst components may vary over a wide range. Suitably the molar ratio of aluminium component to zirconium and/or hafnium component is within the range of 2.5:1 to 50:1, preferably 5:1 to 10:1. The molar ratio of the zirconium and/or hafnium component to the compound of formula (I) is suitably from 0.05:1 to 1:1, preferably from 0.15:1 to 0.25:1.

The catalyst composition as described above has been found to be suitable for use in the oligomerization of ethene. Therefore, according to a further aspect of the present invention there is provided a process for the preparation of substantially linear alpha olefins, comprising reacting ethene, or an ethene-containing gas, under oligomerization conditions in the presence of a catalyst composition as described above.

The oligomerization is preferably carried out at elevated temperatures suitably from 50 to 150 °C, preferably from 100 to 125 °C and at elevated pressure, suitably from 10 to 100 barg, preferably from 30 to 50 barg. The catalyst composition is suitably though not necessarily employed in an inert liquid solvent. Suitable solvents include, for example, chlorobenzene, n-heptane, cyclohexane, toluene, benzene, pentane, hexane, octane, nonane, decane, xylene, dichlorobenzene and dichloroethane. Preferred solvents are chlorobenzene and n-heptane. The oligomerization can be carried out in a batch or continuous operation. Reaction times of from 15 min to 5 hours have been found to be suitable dependent on the activity of the catalyst to give an ethene uptake suitably of 0.5 to 10 kg/g Zr or Hf.h.

After a suitable predetermined reaction time a conventional catalyst deactivating agent, such as n-propanol, may be added to the reaction mixture to terminate the reaction.

The starting material may be pure ethene or a mixture of ethene together with an inert gas, such as nitrogen or helium. The reaction is preferably carried out in the absence of air or moisture.

The product linear alpha olefins suitably have a chain length of from 6 to 20 carbon atoms.

The present invention will now be further described with reference to the following example.

Example

Preparation of Catalyst Compositions

Catalyst liquors A to F were prepared having the following compositions:

Catalyst liquor A
zirconium tetrachloride (0.233g; 1.00 mmol)
diethylaluminium chloride (0.301g; 2.50 mmol)
1,1',3,3'-tetraphenyl-2,2'-biimidazolidinylidene (0.08g; 0.18 mmol)
chlorobenzene (500ml)

Catalyst liquor B

The composition was similar to that of A except that the tetraphenylbiimidazolidinylidene was replaced by 1,1',3,3'-tetra-p-tolyl-2,2'-biimidazolidinylidene (0.1g; 0.20 mmol).

Catalyst liquor C (comparative)

The composition was similar to that of A except that the tetraphenylbiimidazolidinylidene was replaced by 1,2-bis(dimethylamino)-ethane (0.023g; 0.20mmol).

Catalyst liquor D
zirconium tetrachloride (0.233g; 1.00 mmol)
diethylaluminiumchloride (0.301g; 5.00 mmol)
1,1',3,3'-tetraphenyl-2,2'-biimidazolidinylidene (0.04g; 0.1 mmol)
n-heptane (500 ml)

Catalyst liquor E (comparative)

The composition was similar to that of D except that the tetraphenylbiimidazolidinylidene was replaced by triethylamine (0.01g; 0.1 mmol).

Catalyst liquor F (comparative)

The composition was similar to that of E except that no amine was included.

Oligomerization of Ethene

The catalyst liquors A to F described above were employed in the oligomerization of ethene. In each case pressure was maintained at 30 barg by continuous recharging to replace consumed ethene and the temperatures controlled at 100°C by internal cooling. The reaction time is given in Table 1 below. At the end of this time, the reaction was terminated by addition of n-propanol (2ml). The product distribution was determined by gas chromatography and the "K-factor" calculated for each catalyst. The K-factor, which is indicative of the relative proportions of the product alpha-olefins, is the molar ratio of $[C_{n+2}]/[C_n]$ calculated from the slope of the graph of log $[C_n$ mol %$]$ versus n, where n is the number of carbon atoms in a particular alpha-olefin. Lower K-factors indicate a higher proportion of the lower alpha olefins in the range $C_4$-$C_{20}$ in the oligomerization product. The selectivity to lower alpha olefins (LAO Sel.) given in Table 1 is based on the weight percentage of $C_{12}$ linear alpha-olefin in the total $C_{12}$ fraction.

Table 1

| Catalyst Liquor | Reaction Time (min) | Ethene Uptake (kg/g Zr.h) | K factor | LAO Sel. (wt %) |
|---|---|---|---|---|
| A | 30 | 5.3 | 0.70 | 93 |
| B | 30 | 0.8 | 0.56 | 96 |
| C | 30 | 7.0 | 0.70 | 93 |
| D | 30 | 2.0 | 0.67 | 92 |
| E | 60 | 1.2 | 0.64 | 90 |
| F | 40 | 2.9 | 0.67 | 93[1] |

Note 1. A high polymer make was observed in this reaction.

## Claims

1. A catalyst composition comprising a zirconium or hafnium compound, an aluminium compound and a compound of the general formula (I)

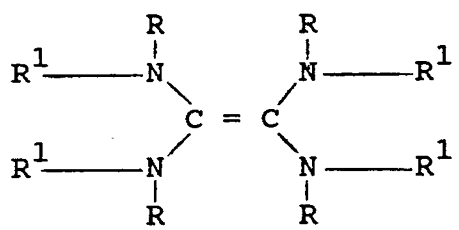

(I)

in which each R, which may be the same or different, represents an optionally substituted alkyl, cycloalkyl, aralkyl or aryl group, or groups R on cis nitrogen atoms may together form an aliphatic, cycloaliphatic or aromatic bridging group which may contain heteroatoms; and each $R^1$, which may be the same or different, represents an optionally substituted alkyl, cycloalkyl, aralkyl or aryl group, or groups $R^1$ on adjacent nitrogen atoms may together form an aliphatic, cycloaliphatic or aromatic bridging group which may contain hetero atoms; or adjacent groups R and $R^1$ together with the nitrogen to which they are attached may form an optionally substituted heterocyclic ring.

2. A catalyst composition according to claim 1 wherein the compound of general formula (I) is selected from compounds of formula II

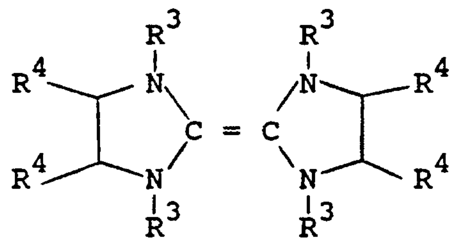

(II)

in which each $R^3$, which may be the same or different, represents an optionally substituted alkyl, cycloalkyl, or aryl group, or groups $R^3$ on different rings may be linked to form an optionally substituted alkylene linkage and each $R^4$, which may be the same or different, represents a hydrogen atom or an optionally substituted alkyl group.

**3.** A catalyst composition according to claim 1 or 2 wherein the zirconium or hafnium compound is an inorganic halide or an alkoxide.

**4.** A catalyst composition according to claim 3 which comprises zirconium tetrachloride.

**5.** A catalyst composition according to any one of the preceding claims wherein the aluminium compound is selected from aluminium trichloride and an organoaluminium halide.

**6.** A catalyst composition according to claim 5 wherein the aluminium compound is diethylaluminium chloride.

**7.** A catalyst composition according to any one of the preceding claims wherein the molar ratio of aluminium component to zirconium or hafnium component is from 2.5:1 to 50:1 and the molar ratio of the zirconium or hafnium component to the compound of formula (I) is from 0.05:1 to 1:1.

**8.** A process for the preparation of substantially linear alpha olefins comprising reacting ethene, or an ethene-containing gas, under oligomerization conditions in the presence of a catalyst composition in accordance with any one of the preceding claims.

**9.** A process according to claim 8 wherein the oligomerization is carried out at a temperature in the range of from 50 to 150 °C, a pressure in the range of from 10 to 100 barg for a time of from 0.25 to 5 hours.

**10.** A process according to claim 8 or 9 wherein the oligomerization is carried out in the presence of a solvent selected from chlorobenzene and n-heptane.

**11.** Substantially linear alpha olefins when obtained by the process of any one of claims 8 to 10.

European Patent Office

# EUROPEAN SEARCH REPORT

Application Number

**EP 90 20 3286**

## DOCUMENTS CONSIDERED TO BE RELEVANT

| Category | Citation of document with indication, where appropriate, of relevant passages | Relevant to claim | CLASSIFICATION OF THE APPLICATION (Int. Cl.5) |
|---|---|---|---|
| D,Y | Angewandte Chemie International Edition vol. 7, no. 10, October 1968, Weinheim DE pages 766 - 779; N. Wiberg: "Tetraaminoethylenes as strong electron donors" * page 767, column 2, paragraph 2 * --- | 1-6,8 | C 07 C 2/30 B 01 J 31/38 |
| Y | DE-A-2 318 953 (ESSO RESEARCH AND ENGINEERING) * claim 1 * --- | 1-6,8 | |
| D,A | EP-A-0 241 596 (IDEMITSU PETROCHEMICAL) * page 3, paragraph 4 * --- | 1 | |
| D,A | EP-A-0 295 960 (EXXON CHEMICAL PATENTS) * page 3, line 56 - page 3, line 58 * --- | 1 | |
| A | US-A-3 855 341 (J.F. MOTIER ET AL) * claim 2 * --- | 1 | |
| A | DE-A-2 063 038 (MITSUBISHI PETROCHEMICAL) * claim 1 * ----- | 1 | |

TECHNICAL FIELDS SEARCHED (Int. Cl.5)

C 07 C
B 01 J

The present search report has been drawn up for all claims

| Place of search | Date of completion of search | Examiner |
|---|---|---|
| Berlin | 05 April 91 | PROBERT C.L. |

CATEGORY OF CITED DOCUMENTS

X: particularly relevant if taken alone
Y: particularly relevant if combined with another document of the same catagory
A: technological background
O: non-written disclosure
P: intermediate document
T: theory or principle underlying the invention

E: earlier patent document, but published on, or after the filing date
D: document cited in the application
L: document cited for other reasons

&: member of the same patent family, corresponding document